# EUROPÄISCHE PATENTSCHRIFT

(11) **EP 2 387 351 B1**
(45) Veröffentlichungstag und Bekanntmachung des Hinweises auf die Patenterteilung: **19.06.2013**
(21) Anmeldenummer: 10700702.3
(22) Anmeldetag: 13.01.2010
(51) Int. Cl.: A61B 5/00, A61B 5/20

(54) **MEDIZINISCHES ÜBERWACHUNGSNETZWERK**
MEDICAL MONITORING NETWORK
RESEAU DE SURVEILLANCE MEDICAL

(30) Priorität: 14.01.2009 EP 09150545
(43) Veröffentlichungstag der Anmeldung: 23.11.2011
(73) Patentinhaber: Roche Diagnostics GmbH, 68305 Mannheim (DE); F. Hoffmann-La Roche AG, 4070 Basel (CH)
(72) Erfinder: RÖSICKE, Bernd, 68305 Mannheim (DE); FROECH, Sybille, 67551 Worms (DE); NIESPOREK, Christian, 69120 Heidelberg (DE); REISER, Wolfgang, 68259 Mannheim (DE)
(74) Vertreter: Stößel, Matthias
(86) Internationale Anmeldenummer: PCT/EP2010/000116
(87) Internationale Veröffentlichungsnummer: WO 2010/081675

(56) Entgegenhaltungen:
- EP-A- 1 062 906
- WO-A-2008/030480
- DE-A1-102005 059 149
- US-A1- 2003 125 017
- US-B1- 6 754 472
- US-B2- 7 163 511

## Beschreibung

### Gebiet der Erfindung

Die Erfindung betrifft ein Netzwerk zur Überwachung von Körperfunktionen eines Patienten, einen Netzwerkknoten zum Einsatz in einem derartigen Netzwerk sowie ein medizinisches System, welches in der Lage ist, ein erfindungsgemäßes Netzwerk einzubinden. Derartige Netzwerke, Netzwerkknoten und medizinische Systeme werden insbesondere eingesetzt, um Patienten mit chronischen Erkrankungen und/oder Risikopatienten, bei welchen mehrere Körperfunktionen gleichzeitig überwacht und/oder beeinflusst werden müssen, zu betreuen.

### Stand der Technik

Sowohl im klinischen Bereich als auch in der privaten Gesundheitsvorsorge besteht in vielen Fällen ein Bedarf an Systemen, welche in der Lage sind, das komplexe Zusammenspiel einzelner Körperfunktionen eines Patienten zu überwachen und ggf. Körperfunktionen gezielt zu beeinflussen. Beispielsweise kann es sich dabei um die Betreuung chronisch kranker Patienten handeln, wie beispielsweise Diabetes-Patienten. Auch Risikopatienten können auf diese Weise betreut werden, beispielsweise Risikopatienten, bei denen bekanntermaßen ein Infarktrisiko in erhöhtem Maße gegeben ist. Allgemein sei darauf hingewiesen, dass der verwendete Begriff "Patient" im Rahmen der vorliegenden Erfindung jedoch den Zielgruppenkreis nicht notwendigerweise auf kranke menschliche oder tierische Patienten beschränkt, sondern dass grundsätzlich auch gesunde Zielgruppen mittels der im Folgenden vorgeschlagenen Vorrichtungen betreut werden können. Allgemein kann daher der Begriff "Patient" zumindest weitgehend mit dem Begriff "Benutzer" gleichgesetzt werden.

Eine Herausforderung bei komplexen medizinischen Systemen stellt in vielen Fällen die Kommunikation zwischen den einzelnen Komponenten des Systems dar. Medizinische Kommunikationssysteme sind aus verschiedenen Schriften des Standes der Technik bekannt. Beispielsweise beschreibt US 7,161,484 B2 ein System zur Überwachung medizinischer Parameter eines Patienten mit mindestens einem Sensor zum Detektieren mindestens eines vorgegebenen medizinischen Parameters. Weiterhin ist ein Übertragungsmittel zum Übertragen der medizinischen Parameter, die der Sensor detektiert hat, vorgesehen, wobei die Übertragung an einen entfernt angeordneten Server erfolgt. US 7,163,511 B1 beschreibt eine Vorrichtung und ein Verfahren zum häufigen Messen der Konzentration eines Analyten in einem biologischen System. Dabei wird ein Überwachungssystem eingesetzt, welches mindestens zwei Komponenten umfasst, um die Datensammlung und das Anzeigen der Daten zu erleichtern.

Drahtlose Kommunikation im näheren Umfeld von Patienten findet heute überwiegend mit Funksystemen statt, die das gesamte elektromagnetische Feld nutzen und üblicherweise im Fernfeld operieren. Bei einer Fernfeldkommunikation ist der Abstand eines Empfängers von einer Senderantenne größer als die doppelte Wellenlänge der gewählten Funk-Trägerfrequenz. Bei 2,45 GHz ist dies ungefähr 0,3 m. Diverse Funktechnologien sind unter IEEE802.11 und verwandten Normen standardisiert. Hauptmerkmale dabei sind, dass eine so genannte ISM-Frequenz (Industry-Sience-Medical, beispielsweise 2,45 GHZ) eingesetzt wird und dass mit begrenzter Sendeleistung von beispielsweise ca. 100 mW Distanzen von ca. 1 - 10 m überbrückt werden. ISM-Frequenzen sind allgemein zugängliche, d.h. nicht durch Organisationen oder Regierungen nach strengen Regeln zugewiesene Frequenzbänder. Als einziges derzeit ohne Einschränkungen weltweit unter Beachtung der geltenden Normen einsetzbares ISM-Frequenzband gilt das 2,45 GHz-Band.

Weiterhin sind Systeme im Einsatz, die nur die magnetische Feldkomponente nutzen. Damit können, physikalisch bedingt, nur Entfernungen innerhalb des Antennen-Nahfeldes überbrückt werden. Solche Systeme sind als RFID-Systeme (Radio Frequency Identification, auch Transponder genannt) oder als NFC-Systeme (Near Field Commmunication) im Einsatz. RFID-Systeme zeichnen sich dadurch aus, dass ein so genannter Leser Daten und Energie in einem so genannten Transponder induziert. Der Transponder modifiziert diese Daten ggf. und gibt diese wieder an den Leser zurück. Der Transponder ist in der Regel nur aktiv, wenn er sich im Einflussfeld der Energie des Lesers befindet. NFC arbeitet mit den gleichen Strukturen und Protokollen wie RFID, wobei hier jedoch auch der Transponder eine eigene Energiequelle umfasst, so dass nur die Kommunikation vom Leser aktiviert wird, die Anwendung aber auch außerhalb des Leser-Einflusses aktiv bleiben kann. Dies ist besonders bei verteilten, kontinuierlich messenden Sensor-Systemen von Vorteil.

Seit einiger Zeit sind auch Kommunikationssysteme bekannt, welche nur den elektrischen Feldanteil des elektromagnetischen Feldes nutzen. Bedingt durch die Durchschlagsfestigkeit von Luft, welche bei ca. 1000 V/mm liegt, kann der elektrische Feldanteil maximal nur ca. 1/90 000 der Energie des magnetischen Feldes übertragen. Der Fernwirkungsanteil ist daher in vielen Fällen auf einen direkten Berührungskontakt begrenzt.

Allerdings hat es sich dabei gezeigt, dass ein menschlicher Körper sich relativ gut zur Leitung dielektrischer Verschiebungsströme eignet. Die Übertragung von Informationen ist daher möglich, ohne dass diese den leitenden Körper weiträumig verlassen. Derartige Netzwerke, welche im Nahfeldbereich arbeiten und den menschlichen Körper zur Übertragung von Signalen nutzen, sind insbesondere im Bereich der Anwendungen für die persönliche Information und Kommunikation bekannt, beispielsweise aus US 6,542,717 B1, aus T. G. Zimmerman: "Personal Area Networks (PAN): Near-Field Intra-Body Communication", Mästerarbeit am Massachusetts Institute of Technology, September 1995 oder aus M. S. Wegmüller: "Intra-Body Communication for Biomedical Sensor Networks", Dissertation, ETH Zürich, 2007. Dort werden derartige Netzwerke auch als PAN (Personal Area Network, Personen Umfeld-Netzwerk) bezeichnet. Derartige Netzwerke setzen elektrische Felder als Kommunikationsmedium zwischen Transmittern ein, welche an Personen angeordnet sind.

Auch aus dem medizinischen Bereich sind Systeme bekannt, welche den menschlichen Körper zur Übermittlung von Signalen nutzen. So beschreibt beispielsweise US 6,315,719 B1 ein System, welches für eine medizinische Langzeitüberwachung eines Patienten, beispielsweise eines Astronauten, eingesetzt werden kann. Dabei ist eine autonome Sensoreinheit an einem Körper eines Menschen angeordnet, welche über Elektroden verfügt. Mittels eines Klebestreifens werden diese Elektroden auf der Haut angeordnet. Weiterhin ist ein am Körper getragener Sender und Empfänger vorgesehen, welcher als zentrale Einheit dient.

Die aus dem Stand der Technik bekannten Kommunikationssysteme, welche sich teilweise grundsätzlich auch für den Einsatz im medizinischen Bereich eignen würden, weisen jedoch eine Reihe von Nachteilen oder technischen Herausforderungen auf. So wird beispielsweise bei einer Fernfeldkommunikation Sendeenergie und damit die modulierte Information räumlich weit gestreut. Durch das Vorhandensein anderer Teilnehmer im gleichen Frequenzband (ISM) wird die Übertragungs-Bandbreite somit eingeschränkt. Das Vorhandensein vieler Teilnehmer im gleichen Frequenzband bedarf komplexer Protokolle, um die Übertragung der Daten zu sichern. So muss einerseits auf die Unversehrtheit der Daten geachtet werden, und es muss andererseits aber auch darauf geachtet werden, dass Daten richtig zugeordnet werden, d.h. dem richtigen Sender bzw. Empfänger. Weiterhin muss ein absichtlicher Datenmissbrauch verhindert werden. Diese Maßnahmen reduzieren insgesamt die Übertragung von Nutzdaten pro Zeiteinheit.

Da die angeführten Funksysteme von zunehmend mehr Anwendungen benutzt werden, ist in Zukunft eine Zunahme der Bandbelegung und damit eine weitere Einschränkung der Bandbreite auf den ISM-Frequenzen zu erwarten. Im Bereich der lebenserhaltenden Diagnostik sind hierfür separate, aber nur für einen sehr eingeschränkten Bereich reservierte Frequenzbänder zugewiesen, beispielsweise der Frequenzbereich WTMS (Wireless Medical Telemetry) bei 402 - 406 MHz, für Intensivstationen in Kliniken oder Ambulanzen. Bei Übertragung diagnostisch relevanter Daten bzw. therapeutischer Anweisungen im nicht lebenserhaltenden Bereich kann es jedoch bei der Funkübertragung in den ISM-Bändern alsbald zu kritischen Latenzzeiten kommen. Dies wäre für ein gekoppeltes Glucose-Insulinsystem nach dem "closed-loop"-Prinzip unter Umständen bereits von Relevanz.

Ferner wird durch die quasi kugelförmige Abstrahlung der Sendenergie bei der Fernfeldkommunikation eine erhebliche Leistung sowohl zum Senden als auch zum Empfangen benötigt, da der Sender stets auf eine maximale Abstrahlungsleistung und der Empfänger stets auf eine maximale Empfangsempfindlichkeit eingestellt sein muss. Beides kostet erhebliche Energie. Eine gerichtete Abstrahlung ist hingegen bei entsprechenden Anwendungen nicht hilfreich, da der Standort der potentiellen Empfänger nicht bekannt ist. Mindestens während der Partner-Suchphase und des Kontaktaufbaus wird daher ein Vielfaches der benötigten Energie in den Raum abgestrahlt. Zumindest bei implantierten Systemen verbietet sich eine derartige Energievergeudung.

Auch sind Freifeldübertragungen mit implantierten Sendern bei höheren Frequenzen in der Regel nicht möglich. Bei einem implantierten Sensor würde eine Sendefrequenz von 2,45 GHz durch die Gewebeflüssigkeit weitgehend absorbiert, da das Absorptionsmaximum von Wasser beispielsweise bei 2,4 GHz liegt. Geeignete, niederfrequente Systeme sind jedoch durch die erforderliche große Antennengröße und geringe Übertragungsbandbreite bezüglich der Applikation begrenzt. Beispielsweise sind Tieridentifikationssysteme für eine relativ niedrige Datenrate bei 125 KHz ausgelegt.

### Aufgabe der Erfindung

Es ist daher eine Aufgabe der vorliegenden Erfindung, Vorrichtungen bereitzustellen, welche die Nachteile bekannter Vorrichtungen vermeiden und welche für die Überwachung von Körperfunktionen eines Patienten eingerichtet sind. Insbesondere sollen die Vorrichtungen in der Lage sein, schnell und zuverlässig Messdaten zu erfassen, um möglichst autonom auf kritische Zustände reagieren zu können.

### Offenbarung der Erfindung

Diese Aufgabe wird durch die Vorrichtungen mit den Merkmalen der unabhängigen Ansprüche gelöst. Vorteilhafte Weiterbildungen der Erfindung, welche einzeln oder in Kombination realisiert werden können, sind in den abhängigen Ansprüchen dargestellt.

Es wird ein Netzwerk zur Überwachung von Körperfunktionen eines Patienten vorgeschlagen. Wie oben dargestellt, muss es sich dem Patienten nicht notwendigerweise um einen kranken Menschen oder ein krankes Tier handeln, sondern es kann eine Überwachung gesunder Patienten erfolgen. Unter einer Überwachung ist allgemein eine Registrierung von Körperzuständen, beispielsweise physiologischen Körperzuständen und/oder anderen Körperzuständen, zu verstehen, welche neben der reinen Erfassung dieser Körperzustände und/oder deren Sammlung bzw. Auswertung, auch alternativ oder zusätzlich, therapeutische, d.h. eingreifende und/oder regulierende, Schritte umfassen kann.

Das Netzwerk umfasst mindestens zwei verschiedene, mit dem Körper des Patienten verbindbare Netzwerkknoten. Unter Netzwerkknoten sind dabei Baugruppen zu verstehen, welche, wie unten näher ausgeführt wird, miteinander kommunizieren und Daten und/oder Befehle austauschen können. Das Netzwerk umfasst vorzugsweise mehr als zwei derartige Netzwerkknoten, beispielsweise drei, vier oder mehr derartiger Netzwerkknoten.

Unter "mit dem Körper des Patienten verbindbar" ist dabei eine Eigenschaft zu verstehen, welche die Anordnung der Netzwerkknoten am Körper und/oder im Körper und/oder in unmittelbarer Nähe des Körpers derart ermöglicht, dass eine Signaleinkopplung und/oder eine Signalauskopplung von Signalen in den bzw. aus dem Körper, beispielsweise dem Körpergewebe und/oder der Körperflüssigkeit, erfolgen kann. Dabei wird für die Datenübertragung ein intrakorporaler Leitmechanismus als Kommunikationsbasis genutzt. Beispielsweise können die Netzwerkknoten zu diesem Zweck entsprechende Elektroden aufweisen, welche für die Einkopplung und/oder Auskopplung der Signale eingesetzt werden können. Beispielsweise können eine oder mehrere Elektrodenflächen vorgesehen sein, welche für die Ein- bzw. Auskopplung direkt oder indirekt mit der Hautoberfläche des Patienten in Verbindung bringbar sind.

Mindestens zwei der Netzwerkknoten, vorzugsweise drei, vier oder mehr der Netzwerkknoten, insbesondere alle der Netzwerkknoten, weisen dabei jeweils mindestens eine medizinische Funktion auf. Unter einer medizinischen Funktion kann dabei grundsätzlich eine beliebige pharmazeutische, diagnostische, analytische, therapeutische, chirurgische, medikative oder regulatorische Funktion oder eine Kombination der genannten und/oder anderer Kombinationen verstanden werden, welche direkt oder indirekt mit Körperfunktionen des Patienten in Wechselwirkung stehen. Insbesondere kann es sich dabei um eine diagnostische Funktion und/oder eine Medikationsfunktion handeln. Beispiele werden unten aufgezählt.

Die Netzwerkknoten sind, wie oben dargestellt, eingerichtet, um über den Körper des Patienten unmittelbar miteinander zu kommunizieren und Daten und/oder Befehle auszutauschen. Unter einer unmittelbaren Kommunikation wird dabei eine Kommunikation verstanden, bei welcher nicht notwendigerweise auf ein externes, außerhalb des Körpers des Patienten angeordnetes, zentrales Kommunikationsgerät zurückgegriffen werden muss. Die Netzwerkknoten bilden vorzugsweise gemeinsam ein Nahfeld-Netzwerk innerhalb des Körpers, über welches diese miteinander kommunizieren und Daten und/oder Befehle austauschen können. So lassen sich beispielsweise, durch deren Integration in entsprechenden Netzwerkknoten, verschiedene am und/oder im Körper eingesetzte Geräte und/oder Sensoren zu einem Netzwerk verbinden, welche einerseits Daten austauschen können, andererseits aber auch aufgrund dieser und/oder anderer Daten eine Steuerung vornehmen können, beispielsweise eine Steuerung implantierter Geräte. Dabei kann der Austausch der Daten und/oder Befehle jeweils über den Körper erfolgen, so dass ein intrakorporales Nahfeld-netzwerk (near field intrabody network) entsteht.

Die vorliegende Erfindung verknüpft und kombiniert damit die verschiedenen Problemkreise einer komplexen Healthcare-Anwendung mit den spezifischen Vorteilen der verschiedenen Vernetzungsprinzipien bzw. Vernetzungstechnologien für ein körpernahes Netzwerk, d.h. ein Netzwerk am Körper, im Körper oder in nahräumlicher Entfernung zum Körper. Im Nachfolgenden werden solche Problemkreise exemplarisch näher erläutert. Je nach Applikation können die beschriebenen Elemente auch kombiniert werden, so dass bestimmte Problemkreise gezielt adressiert werden können.

Mindestens einer der Netzwerkknoten kann insbesondere einen Sensor umfassen, also ein Element zur qualitativen und/oder quantitativen Erfassung mindestens einer Messgröße, beispielsweise einer physikalischen und/oder chemischen Messgröße. Insbesondere kann mindestens einer der Netzwerkknoten mindestens einen der folgenden Sensoren umfassen: einen Sensor zur Erfassung mindestens eines Analyten in einer Körperflüssigkeit, insbesondere Glukose, Lactat, CO₂, HB, HB-O₂; einen Sensor zur Erfassung mindestens einer Körperfunktionen, insbesondere einer Nierenfunktion; einen Blutdrucksensor; ein Oxymeter; einen Pulsmesser; einen Bewegungsmelder; einen Temperatursensor. Auch Kombinationen der genannten und/oder anderer Arten von Sensoren können in einem oder in mehreren Netzwerkknoten umfasst sein.

Insbesondere können mindestens einer der Netzwerkknoten auch mindestens einen ganz oder teilweise in den Körper des Patienten implantierbaren Sensor umfassen. Dieser Sensor kann eingerichtet sein, um mindestens eine Messgröße in einem Körpergewebe und/oder in einer Körperflüssigkeit zu erfassen, insbesondere eine Konzentration mindestens eines Analyten. Als Beispiele sind auch hier wiederum Glucose und/oder Lactat zu nennen.

Alternativ oder zusätzlich kann mindestens einer der Netzwerkknoten mindestens einen Aktor umfassen, also ein Element, welches mindestens ein Signal ausgibt und/oder mindestes eine Wirkung in einem anderem Element und/oder dem Körper hervorruft. Beispielsweise können einer oder mehrere der folgenden Aktoren umfasst sein: ein Aktor zur Beeinflussung mindestens einer Körperfunktion, insbesondere ein elektrischer Aktor und/oder ein mechanischer Aktor; ein Ventil, insbesondere ein Ventil für eine Harnsteuerung; ein Medikationsaktor, insbesondere eine Medikationspumpe. Auf diese Weise können mittels des mindestens einen Aktors gezielt Körperfunktionen direkt oder indirekt beeinflusst werden und/oder andere Elemente gesteuert werden.

Weiterhin kann das Netzwerk, insbesondere mindestens einer der Netzwerkknoten, mindestens einen Datenspeicher aufweisen, insbesondere einen flüchtigen und/oder nichtflüchtigen Datenspeicher. Der Netzwerkknoten kann dann eingerichtet sein, um eine Datenerfassung von Daten dieses Netzwerkknotens, beispielsweise eines Sensors dieses Netzwerkknotens und/oder von anderen Netzwerkknoten, vorzunehmen.

Beispielsweise kann im Rahmen der vorliegenden Erfindung eine Verbindung mit mindestens einem Sensor für eine kontinuierliche Überwachung ("continuous monitoring sensor") und einer Medikationspumpe, beispielsweise einer Insulinpumpe, und ferner einem Monitoring-System auf der Basis der oben genannten "near field intrabody communication" realisiert werden. Die Netzwerkknoten können dann so das genannte intrakorporale Nahfeldnetzwerk bilden.

Der mindestens eine Sensor kann beispielsweise teilweise oder vollständig implantiert sein und kann beispielsweise in kurzen Abständen kontinuierlich oder diskontinuierlich Messdaten sammeln. Beispielsweise können Messdaten über Glucose in einem Interstitium und/oder im Vollblut (beispielsweise aus Venen oder Arterien) gesammelt werden. Diese Daten können insgesamt gesammelt, ggf. aufbereitet und ggf. in eine geeignete Form umgewandelt werden, aktiv zu den entsprechenden Adressaten im Netzwerk gesendet werden und von dort, beispielsweise nach Anforderung, abgerufen werden.

Der Glucosesensor kann, wie oben dargestellt, alternativ oder zusätzlich durch weitere Sensoren für andere Arten von Parametern ersetzt bzw. ergänzt werden. So können physikalische Parameter gemessen werden, wie beispielsweise Blutdruck, Herzfrequenzen oder Temperatur oder Kombinationen der genannten und /oder anderer Parameter. Alternativ oder zusätzlich lassen sich auch chemische Parameter messen, beispielsweise Blut, Sauerstoff und/oder weitere Analyten. Die Sensoren können vorzugsweise alle spezifischen Daten erfassen, diese optional bereits vor Ort in geeigneter Form aufbereiten und diese dann ebenfalls optional, in einer für eine Kommunikation geeignete Form zwischenspeichern. Beispielsweise kann dies eine für eine asynchrone Kommunikation geeignete Form sein.

Das Netzwerk ist derart eingerichtet sein, dass mindestens zwei und bevorzugt mehrere oder alle der Netzwerkknoten eingerichtet sind, um eine Steuerung des Netzwerkes zu übernehmen. Beispielsweise kann dies eine Steuerung als "Master" des Systems sein. So können beispielsweise einer, mehrere oder alle der Netzwerkknoten als "Master" konfigurierbar sein und dadurch mit einer Koordinationsaufgabe für das gesamte Netzwerk beauftragt werden. Beispielsweise kann ein Netzwerkknoten diese Master-Aufgabe übernehmen, welcher auch über eine Anzeigefunktion verfügt. Auch ein Netzwerkknoten, welcher über eine andere Art der Mensch-Maschine-Schnittstelle verfügt, beispielsweise entsprechende Eingabe-Ausgabe-Mittel, kann für diesen Zweck eingesetzt werden, beispielsweise mindestens ein Vibrator und/oder akustischer Geber.

Das Netzwerk kann auch eingerichtet sein, um geschlossen, d.h. ohne die Notwendigkeit eines Eingriffs von außen, Steuer- und/oder Regelaufgaben zu übernehmen. Beispeisweise kann mindestens einer der Netzwerkknoten mindestens einen Sensor zur Erfassung mindestens einer Messgröße des Patienten umfassen. Mindestens ein anderer der Netzwerkknoten kann dann mindestens eine therapeutische Vorrichtung umfassen, insbesondere eine Medikationsvorrichtung. Das Netzwerk kann eingerichtet sein, um über den Körper des Patienten, also über das intrakorporale Netzwerk, die therapeutische Vorrichtung entsprechend der Messgröße zu steuern und/oder zu regeln. Die Steuerfunktion und/oder Regelfunktion kann dabei von einem oder mehreren der Netzwerkknoten übernommen werden. Beispielsweise kann diese von dem Netzwerkknoten, welcher auch den Sensor umfasst, übernommen werden und/oder von dem Netzwerkknoten, welcher die therapeutische Vorrichtung umfasst. Auch eine Verteilung der Steuer- und/oder Regelfunktionen ist möglich. Es können auch zwischen den Netzwerkknoten spezifische Kommunikationsprofile vereinbart werden. Beispielsweise können derartige spezifische Kommunikationsprofile zwischen einem Glucosesensor und einer Insulinpumpe vereinbart werden. Auch so kann ein geschlossener Regelkreis erzeugt werden. Insgesamt kann diese geschlossene Regel- und/oder Steuerfunktion innerhalb des Netzwerkes derart erfolgen, dass weder ein Eingriff des Patienten von außen erforderlich ist noch dass der Patient über diese Abläufe stets informiert werden muss. So können beispielsweise nur noch Ergebnisse, Statusinformationen oder akute Alarme dem Patienten zur Kenntnis gebracht werden.

Die Netzwerkknoten können insbesondere eingerichtet sein, um über eine asynchrone Datenübertragung miteinander zu kommunizieren. Die Steuerung und Koordination eines intrakorporalen Netzwerkes kann ein speziell an die Belange eines derartigen intrakorporalen Netzwerks angepasstes Protokoll voraussetzen. Bei einer asynchronen Datenübertragung, beispielsweise nach dem Standard UIT 34.13, können die zu übermittelnden Zeichen asynchron, d.h. in der Regel zu beliebigen Zeiten, übertragen werden. Dabei erfolgt ein ungefährer Gleichlauf zwischen dem jeweiligen Sender und dem Empfänger in der Regel nur für die Dauer eines Zeichens. Da die Anforderungen an die Qualität des Gleichlaufs zwischen Sender und Empfänger geringer sind, kann dieser schneller erreicht werden.

Allgemein kann erfindungsgemäß dem Umstand Rechnung getragen werden, dass binnen kurzer Zeit frei verfügbare Funkfrequenzen mit Standardapplikationen überfüllt sein werden. Dementsprechend können in naher Zukunft bei zeitkritischen Medizinapplikationen Probleme auftreten, insbesondere hinsichtlich einer zeitnahen Datenkommunikation. Die vorliegende Erfindung kann, beispielsweise durch die oben beschriebenen Ausgestaltungen, solche Zeitkonflikte drastisch reduzieren und auch zukünftig zunehmend komplexere Datenkommunikationen und Datenstruktuten im körpernahen Umfeld ermöglichen.

Durch das geringe extrakorporale Störpotenzial bei der Kommunikation können solche Systeme auch in kritischen Bereichen eingesetzt werden, wie beispielsweise in einem Emergency Room, einer Intensivstation, in explosionsgeschützten Bereichen oder Ähnlichem.

Weiterhin kann mindestens einer der Netzwerkknoten auch eingerichtet sein, um eine Failsafe-Funktion durchzuführen. Unter einer derartigen Failsafe-Funktion kann eine selbständige Erkennung von abnormalen Zuständen und ggf. eine entsprechende Reaktion auf diese abnormalen Zustände verstanden werden. Beispielsweise kann stets eine Plausibilitätsbetrachtung von übermittelten Daten, Befehlen oder Messwerten erfolgen. Werden beispielsweise vereinbarte Fehlerdiskriminierungsgrenzen überschritten bzw. werden als "normal" eingestufte Bereiche verlassen, so kann hieraus auf das Vorliegen eines abnormalen Zustandes oder Fehlers geschlossen werden. Dann können direkte Maßnahmen eingeleitet werden. Beispielsweise kann der Netzwerkknoten eingerichtet sein, um bei Erkennung eines Fehlerzustandes mindestens eine Fehlerroutine durchzuführen. Beispielsweise kann eine derartige Fehlerroutine ein Abschalten einer Versorgungsspannung beinhalten, um vor biologisch kritischen Gefährdungen zu schützen. Auch andere Fehlerroutinen sind grundsätzlich möglich. Weiterhin ist es, alternativ oder zusätzlich, auch möglich, dass mindestens ein Netzwerkknoten eingerichtet ist, um einen Alarm an den Patienten auszugeben, insbesondere im Fall einer Fehlfunktion des Netzwerkes und/oder im Fall eines Auftretens abnormaler Körperfunktionen.

In einer weiteren bevorzugten Ausgestaltung kann mindestens einer der Netzwerkknoten eine Anzeigevorrichtung umfassen. Insbesondere kann diese Anzeigevorrichtung eine an einem Handgelenk des Patienten tragbare Anzeigevorrichtung, insbesondere eine in einer Armbanduhr integrierte Anzeigevorrichtung, umfassen. Das Handgelenk kann dabei als Schnittstelle zwischen dem Netzwerkknoten mit der Anzeigevorrichtung und dem Körper des Patienten fungieren, da durch die Armbanduhr, ggf. mittels geeigneter Elektroden, ein unmittelbarer Kontakt hergestellt werden kann. Alternativ oder zusätzlich zu einer Anzeigevorrichtung kann mindestens ein Netzwerkknoten, auch wie oben dargestellt, mindestens eine andere Ein- und/oder Ausgabevorrichtung umfassen, so dass ein Patient und/oder ein Arzt unmittelbar Zugriff auf das Netzwerk nehmen kann.

Auch eine oder mehrere weitere Schnittstellen können umfasst sein, um beispielsweise mit anderen, nicht im Netzwerk umfassten Komponenten kommunizieren zu können. Diese Schnittstelle kann grundsätzlich beispielsweise eine drahtgebundene und/oder eine drahtlose Schnittstelle umfassen. Insbesondere kann mindestens einer der Netzwerkknoten zusätzlich für eine Kommunikation außerhalb des Körpers eingerichtet sein, insbesondere eine Fernfeldkommunikation. So können einer oder mehrere der Netzwerkknoten auch derart erweitert werden, das sie über eine Fernfeldkommunikation verfügen, so dass sie Informationen beispielsweise zu Kommunikationsnetzen (beispielsweise BT, WLAN, GSM) und/oder Computernetzwerken weiterereichen können. Dort können dann beispielsweise Daten weiter gesammelt, verdichtet und verhandelt werden. Ein derartiger Datentransfer aus dem Netzwerk heraus wird auch als Upload bezeichnet. Es können auf diesem Wege aber auch Anweisungen und Daten an die Netzwerkknoten, also in das Netzwerk hinein, weitergeleitet werden, was auch als Download bezeichnet werden kann. Vorzugsweise wird der mindestens eine Fernfeldkommunikationsknoten in einem Anzeigegerät an der Körperoberfläche liegen. Allgemein können Kommunikationsknoten voll implantiert sein und/oder auf der Körperoberfläche angeordnet sein und/oder auch entfernt von der Körperoberfläche angeordnet sein.

Ein weiterer Aspekt der vorliegenden Erfindung liegt in einer Energieversorgung eines einzelnen Netzwerkknotens, mehrerer Netzwerkknoten oder des gesamten Netzwerkes. Im Gegensatz zur RFID-Technologie soll das erfindungsgemäße Netzwerk, welches beispielsweise über eine kapazitive Kopplung mit dem Körper funktioniert, aufgrund der in der Regel geringen Energiekopplung vorzugsweise über mindestens eine separate Energieversorgung verfügen. Dies kann beispielsweise in Form von integrierten, primären Batterien und/oder Sekundärbatterien erfolgen und/oder anderen Arten von elektrischen Energiespeichern. Dies erfordert jedoch in regelmäßigen oder unregelmäßigen Abständen Maßnahmen des Trägers, insbesondere hinsichtlich eines Wechsels und/oder Nachladens der elektrischen Energiespeicher. Insbesondere im Fall von Implantaten sind hierbei jedoch aufwändige Eingriffe nötig. Ein Nachladen kann durch einen Kontakt und/oder durch Anlegen eines externen magnetischen Wechselfeldes nicht-invasiv erfolgen.

Alternativ oder zusätzlich ist es jedoch möglich, dass das Netzwerk auch die körpereigene Energiequelle und/oder die Umgebung des Netzwerks als Energiequelle nutzt. Dementsprechend kann mindestens einer der Netzwerkknoten eingerichtet sein, um dem Körper und/oder einer Umgebung des Körpers Energie zu entziehen und diese für eine Energieversorgung und/oder eine Energieversorgung anderer Netzwerkknoten bzw. des gesamten Netzwerkes zu nutzen. Die Energie kann dabei dem Körper und/oder der Umgebung des Körpers auf verschiedene Weise entzogen werden. Beispeisweise kann thermische Energie entzogen werden. Auch Vibrationsenergie kann entzogen werden, beispielsweise mittels entsprechender piezo-elektrischer Generatoren.

Alternativ oder zusätzlich kann der entsprechende Netzwerkknoten jedoch auch eine elektrochemische Energiequelle ausnutzen. Beispielsweise kann eine elektrochemische Energiegewinnung aus Glucose erfolgen, beispielsweise direkt aus der umgebenden Glucose eines implantierten Sensors. Dies erfolgt vorzugsweise derart, dass die Energieentnahme in keinem Fall den Messwert beeinflusst, beispielsweise durch Verarmung der Glukose im Bereich des Messorts. So kann der Netzwerkknoten beispielsweise mindestens einen elektrochemischen Sensor umfassen, wobei der elektrochemische Sensor eingerichtet ist, um in einem Sensormodus mindestens eine Messgröße zu erfassen und wobei der elektrochemische Sensor weiterhin eingerichtet ist, um in mindestens einem Energiegewinnungsmodus elektrochemische Energie zu gewinnen. Beispielsweise kann zwischen den genannten Modi des Sensors hin- und hergeschaltet werden. In gewissem Umfang sind jedoch auch eine gleichzeitige Ausübung und/oder eine zeitliche überlappende Ausübung der beiden Modi möglich.

Hohe Feldstärken und damit ggf. gefährliche Spannungen sollen in dem erfindungsgemäßen Netzwerk nach Möglichkeit vermieden werden. So sollten die auftretenden Schutzkleinspannungen vorzugsweise kleiner als 42 V sein. Zur Erlangung eines hinreichend guten Signal-Störabstandes werden vorzugsweise spezifische, sichere Kommunikationsprotokolle eingesetzt, da insbesondere die Datenübertragungsrate bei Körpernetzwerken in der Regel niedrig ist. Somit lässt sich ein Großteil der verfügbaren Bandbreiten für eine Redundanz und damit für eine Datensicherheit einsetzen.

Mit dieser Überlegung kann auch dem Umstand Rechnung getragen werden, dass ein vorgeschlagenes Netzwerk auch im Falle eines intensivinvasiven Eingriffs am Körper diagnostische und/oder therapeutische Apparate nicht stören sollte.

Das Netzwerk sollte insbesondere flexibel ausgestaltet sein, da die medizinischen Funktionen des Netzwerkes in der Regel individuell auf einzelnen Patienten angepasst sein sollten. So ist es besonders bevorzugt, wenn einzelne Netzwerkknoten beliebig aus dem System herausgenommen bzw. in das System ergänzt werden können. Das Netzwerk kann eingerichtet sein, um neue Netzwerkknoten, vorzugsweise automatisch, zu erkennen und in das Netzwerk einzubinden.

Beispielsweise kann das Netzwerk mindestens ein in das Netzwerk integrierbares und aus dem Netzwerk auch abkoppelbares tragbares Handgerät mit mindestens einer Anzeigefunktion umfassen. Insbesondere kann dieses tragbare Handgerät ein medizinisches Messgerät sein, beispielsweise ein Blutglucose-Messgerät. Alternativ oder zusätzlich kann das Handgerät auch mindestens ein Mobiltelefon, d.h. ein für eine mobile Datenübertragung eingerichtetes Gerät, umfassen. Das Netzwerk kann dementsprechend eingerichtet sein, um bei einem Kontakt des Handgerätes mit dem Körper des Patienten, also einer Stellung, welche eine Signaleinkopplung in den Körper und/oder eine Signalauskopplung aus dem Körper ermöglicht, insbesondere einen Kontakt mit einer Hand des Patienten, das Handgerät automatisch in das Netzwerk einzubinden.

Es ist sogar eine aktive temporäre Einbindung eines Netzwerkes gemäß einem oder mehreren der oben beschriebenen Ausführungsformen in ein übergeordnetes medizinisches System möglich. Dementsprechend wird weiterhin ein medizinisches System vorgeschlagen, beispielsweise ein Operationssystem und/oder ein Intensivmedizinisches System und/oder ein Anästesie-System. Dieses medizinische System kann mindestens eine Kommunikationsvorrichtung umfassen, welche eingerichtet ist, um eine Gegenwart eines Netzwerkes gemäß der vorliegenden Erfindung, also eines "body area networks" (BAN) gemäß der obigen Beschreibung, zu erkennen, vorzugsweise automatisch. Die Kommunikationsvorrichtung kann weiterhin eingerichtet sein, um mit dem Netzwerk zu kommunizieren, und somit dieses in das medizinische System einzubinden. Diese Kommunikation kann insbesondere über eine Fernfeldkommunikation erfolgen. Das medizinische System kann dann eingerichtet sein, um mit dem Netzwerk Daten und/oder Befehle auszutauschen.

Neben dem Netzwerk und dem medizinischen System wird weiterhin ein Netzwerkknoten zum Einsatz in einem Netzwerk gemäß einer oder mehrerer der oben beschriebenen Ausführungsformen vorgeschlagen. Der Netzwerkknoten kann mindestens eine medizinische Funktion umfassen, insbesondere eine diagnostische Funktion und/oder eine Medikationsfunktion. Diesbezüglich kann insbesondere auf die obige Beschreibung der möglichen Ausgestaltungen der Netzwerkknoten verwiesen werden. Der Netzwerkknoten umfasst weiterhin mindestens eine mit dem Körper des Patienten verbindbare Kommunikationseinheit und ist eingerichtet, um über den Körper des Patienten unmittelbar mit anderen Netzwerkknoten des Netzwerkes zu kommunizieren und Daten und/oder Befehle auszutauschen. Für weitere mögliche Ausgestaltungen kann auf die obige Beschreibung verwiesen werden.

Das vorgeschlagene Netzwerk, der vorgeschlagene Netzwerkknoten und das vorgeschlagene medizinischen System weisen gegenüber bekannten, ähnlichen Vorrichtungen einige Vorteile auf. So lassen sich neue diagnostische Methoden durch eine vereinfachte Vernetzung von relevanten intrakorporalen Parametern (innerhalb des BAN) und extrakorporalen Parametern (beispielsweise innerhalb eine Fernfeldes) mittels der Netzwerkknoten realisieren. Durch ein vorzugsweise permanentes Vernetzen der Parameter kann eine präzisiere diagnostische Aussage und/oder eine ggf. verbesserte Therapie ermöglicht werden. Weiterhin ist das vorgeschlagene Netzwerk mit geringem Aufwand an die persönliche Patientensituation anpassbar. Dies kann beispielsweise hinsichtlich der zu verwendenden Parameter, hinsichtlich der räumlichen Anordnung und/oder hinsichtlich der zeitlichen Ausgestaltung von Messungen und/oder sonstigen medizinischen Maßnahmen erfolgen.

Weiterhin kann durch angepasste Leitungsmechanismen zur Übertragung der Daten erreicht werden, dass diese Daten nur minimal räumlich gestreut werden. Durch ein Vermeiden unnötiger Streuungen des räumlichen Datenverkehrs kann sowohl eine Zunahme der persönlichen Datensicherheit als auch eine Abnahme von Datenfehlern, insbesondere durch Kollisionen, realisiert werden.

Durch eine Verbindung logischer und ergonomischer Handlungen durch den Patienten mit entsprechenden Funktionsabläufen sind Vereinfachungen bei der Initialisierung und Konditionierung und der Kalibration der vorgeschlagenen Netzwerke möglich. Beispielsweise lässt sich für eine Glucosemessung im Interstitium für eine Kalibration ein Vollblutmesswert automatisch von einem Blutglucose-Meter in der Hand des Patienten an einen Langzeit-Sensor ("continuous monitoring sensor") leiten.

Durch eine spezifische Vernetzung kann beispielsweise für die Knoten im Netzwerk eine wesentlich geringere Energie benötigt werden als im Freifeld. Das System wird damit insgesamt energieeffizienter, und es lassen sich Handhabungsschritte zur Energiebeschaffung durch den Patienten vermeiden. Hierdurch wird auch eine flexible, dezentrale Energiekonzeption möglich. Einzelne Netzwerkknoten können auch, da wenig Energie für die Kommunikation benötigt wird, diese Energie sogar aus dem direkten Umfeld beziehen, beispielsweise einem Glucosesensor.

Weiterhin lässt sich das Netzwerk auch auf einfache und flexible Weise an die geforderten Rahmenbedingungen anpassen. Beispielsweise können Befindlichkeitsprofile durch die einfache Vernetzung spezifisch überwacht werden. Hierdurch kann ein umfassendes Healthcare-Management erfolgen und/oder ein Management beim Leistungssport.

Durch reduzierte Feldstärken bei der intrakorporalen Leitung ist der Betrieb der Netzwerke auch in kritischen Umgebungen möglich, beispielsweise in Intensivstationen, in einem Emergency-Room, in explosionsgefährdeten Bereichen (beispielsweise in der Umgebung von Tankstellen) oder in einem Flugzeug. Die intrakorporalen Netzwerke der vorgeschlagenen Art können temporär sogar als Bestandteile umfangreicherer, intensivdiagnostischer Systeme fungieren und somit beispielsweise Unterstützung bei Operationen und/oder in der Anästhesie leisten.

Vorteile ergeben sich weiterhin aus der jeweils günstigen Anbindung der einzelnen Komponenten des Netzwerkes und/oder des medizinischen Systems. So lassen sich diese Komponenten jeweils mit der für die verschiedenen Anforderungen optimalen Netztechnik miteinander verknüpfen. Beispielsweise lassen sich Sensoren und/oder Aktoren untereinander über das BAN, also das Netzwerk, verbinden, während eine Anbindung des gesamten Netzwerkes und/oder einzelner Komponenten des Netzwerkes an übrige Bestandteile des medizinischen Systems, beispielsweise über Mobilfunk und/oder anderer Arten von Fernfeldkommunikationen, erfolgen kann. Weiterhin kann erfindungsgemäß dem Umstand Rechnung getragen werden, dass Fernfeld-Frequenzbänder in der Regel ein Kapazitätsproblem aufweisen bzw. in naher Zukunft ein derartiges Kapazitätsproblem aufzeigen werden.

Weiterhin sind mit dem vorgeschlagenen Netzwerk selbstlernende, organisierende Netze denkbar. Beispielsweise kann bei einem Berühren eines Netzwerkknoten durch einen Benutzer eine Zuordnung des Netzwerkknotens zum Benutzer erfolgen. Nach dem Ansetzen des Netzwerkknotens können sich dann die Netzwerkknoten untereinander verständigen und beispielsweise Modalitäten der weiteren Zusammenarbeit im Netzwerk austauschen.

Die genannten optionalen Failsafe-Konzepte können ebenfalls dem Netzwerkgedanken Rechnung tragen. So können beispielsweise einzelne Netzwerkknoten bei definierten Situationen autark Entscheidungen treffen und Maßnahmen durchführen. Beispielsweise kann ein "continuous monitoring sensor" einen Austritt von Substanzen feststellen, beispielsweise einen Austritt von Elektrodenmaterial und/oder anderer Komponenten von Sensoren. Beispielsweise kann ein Kupferaustritt aus einer Elektrode und/oder Zuleitung festgestellt werden. Wird ein Austritt festgestellt, so können beispielsweise entsprechende Maßnahmen eingeleitet werden, beispielsweise eine Stromunterbrechung. Auch Failsafe-Strategien mit weiteren Netzwerkknoten im Netzwerk können organisiert werden, auch in einer selbstorganisierenden Weise. Auf diese Weise kann die Failsafe-Modalität auf das Gesamtnetz ausgedehnt werden. Es können somit also mehrere Netzwerkknoten an der mindestens einen Failsafe-Funktion beteiligt werden.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen zwei gleiche oder funktionsgleiche bzw. hinsichtlich ihrer Funktionen entsprechende Elemente.

Im Einzelnen zeigt:
- Figur 1: Eine Prinzipdarstellung einer nicht-erdbezogenen intrakorporalen Nahfeldkommunikation;
- Figur 2: ein Ausführungsbeispiel eines intrakorporalen Netzwerkes im Diabetesumfeld;
- Figur 3: eine Prinzipskizze einer Signalgewinnung und einer Betriebsenergiegewinnung aus derselben Quelle; und
- Figur 4: eine Prinzipskizze einer Energiegewinnung aus einer separaten Quelle.

### Ausführunsgbeispiele

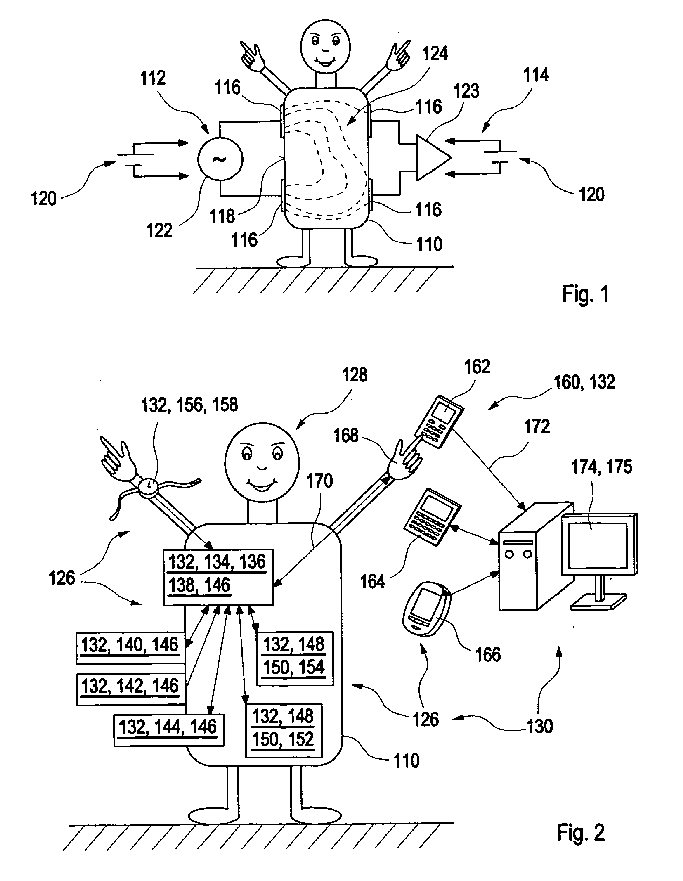

In Figur 1 ist eine Prinzipdarstellung einer Signalübertragung über einen Körper 110 von einem Sender 112 zu einem Empfänger 114 dargestellt. Sender 112 und Empfänger 114 verfügen dabei jeweils über Elektroden 116, welche unmittelbar auf eine Hautoberfläche 118 aufgebracht werden oder in unmittelbarer Nähe dieser Hautoberfläche 118 angeordnet sind. Sowohl Sender 112 als auch Empfänger 114 verfügen jeweils über eine Energiequelle 120. Diese Energiequelle 120 kann beispielsweise mindestens einen Energiespeicher, beispielsweise eine Batterie und/oder einen Akkumulator, und/oder einen Energieerzeuger umfassen. Im Sender 112 wird aus dieser Energiequelle 120 ein Signalgenerator 122 gespeist, welcher die Elektroden 116 des Senders 112 beispielsweise mit einer Wechselspannung beaufschlagt. Hierdurch entsteht im Körper 110 ein elektrisches Feld 124, welches zur intrakorporalen Nahfeld-Übertragung eingesetzt werden kann. Der Empfänger 114 kann neben der Energiequelle 120 und den Elektroden 116 beispielsweise weiterhin über einen oder mehrere Verstärker 123 verfügen, um von den Elektroden 116 aufgenommene Signale zu verstärken und ggf. ganz oder teilweise zu verarbeiten. Weiterhin können der Sender 112 und der Empfänger 114 weitere, in Figur 1 nicht dargestellte Bestandteile umfassen, wie beispielsweise Datenverarbeitungsgeräte und/oder Geräte zur Signalverarbeitung.

Das Prinzip der intrakorporalen Datenübertragung ist aus dem Stand der Technik, beispielsweise dem oben beschriebenen Stand der Technik, grundsätzlich bekannt. Beispielsweise kann auf die oben zitierte Dissertation von M. S. Wegmüller oder die Masterarbeit von T. G. Zimmerman verwiesen werden. Die dort dargestellten Prinzipien und Methoden der intrakorporalen Datenübertragung können grundsätzlich auch im Rahmen der vorliegenden Erfindung eingesetzt werden, beispielsweise die dargestellten Prinzipien zur Einkopplung und/oder Auskopplung von Signalen und/oder der Signalverarbeitung. Dargestellt ist in Figur 1 nur das Grundprinzip einer nicht-erdbezogenen intrakorporalen Nahfeldkommunikation, welches hier nur exemplarisch als bipolare Punkt-zu-Punkt-Verbindung zwischen Sender 112 und Empfänger 114 gezeigt ist. Alternativ oder zusätzlich sind jedoch grundsätzlich auch erdbezogene intrakorporale Nahfeldkommunikationen möglich. Auch komplexere Ausgestaltungen sind grundsätzlich möglich. So können prinzipiell beliebige Sender-Empfänger-Knoten auf dem Körper angebracht sein. Die Sender 112 können prinzipiell auch als Empfänger 114 fungieren und umgekehrt.

Ausgehend von diesem bekannten Grundprinzip ist in Figur 2 ein Ausführungsbeispiel eines erfindungsgemäßen Netzwerkes 126 zur Überwachung von Körperfunktionen eines Körpers 110 eines Patienten 128 dargestellt, sowie ein Ausführungsbeispiel eines erfindungsgemäßen medizinischen Systems 130, in welches das Netzwerk 126 eingebunden ist. Exemplarisch ist hier ein Netzwerk 126 dargestellt, welches im Diabetes-Umfeld eingesetzt werden kann. Grundsätzlich sei darauf hingewiesen, dass auch andere Arten von Körperfunktionen eines Patienten überwacht werden können, dass auch andere Arten von Krankheitsbildern überwacht werden können und/oder auch andere Arten von Gesundheitszuständen, und dass grundsätzlich, wie oben beschrieben, der Begriff eines Patienten 128 allgemein als menschliche oder tierische Person zu verstehen ist, ohne Beschränkung auf Anwender oder Kunden mit abnormalen Körperfunktionen.

Das Netzwerk 126 umfasst eine Mehrzahl von Netzwerkknoten 132. In diesem Ausführungsbeispiel ist das Netzwerk 126 als sternförmiges Netzwerk ausgestaltet und umfasst als zentralen Netzwerkknoten 132, der auch als Master-Netzwerkknoten 134 fungieren kann, einen Netzwerkknoten 132 mit einem Glucosesensor 136. Dieser Glucosesensor 136 ist im vorliegenden Ausführungsbeispiel vorzugsweise als implantierbarer Sensor 138 ausgestaltet, insbesondere als Langzeitsensor oder "continuous monitoring sensor", welcher zumindest teilweise in einem Körpergewebe des Patienten 128 implantiert werden kann. Dieser Master-Netzwerkknoten 134 umfasst, wie vorzugsweise auch alle anderen Netzwerkknoten 132, neben dem Glucosesensor 136 zudem vorzugsweise mindestens einen Sender 112 und mindestens einen Empfänger 114, welche auch, zumindest teilweise, bauteilidentisch ausgestaltet sein können. Beispielsweise können zu diesem Zweck wiederum eine, zwei oder mehr Elektroden 116 vorgesehen sein, analog zur Prinzipdarstellung in Figur 1. Diese Sender 112 und Empfänger 114 sowie die zugehörigen Elektroden 116 sind in der Darstellung gemäß Figur 2 nicht gezeigt und können jeweils optional hinzugefügt werden.

Neben dem Master-Netzwerkknoten 134 umfasst das Netzwerk 126 eine Mehrzahl von weiteren Netzwerkknoten 132, welche auch optional austauschbar sind. So kann beispielsweise ein Temperatursensor 140 als Netzwerkknoten 132 vorgesehen sein, beispielsweise ein Infrarot-Temperatursensor, ein Hautkontakt-Temperatursensor, ein implantierter und/oder implantierbarer Temperatursensor oder Ähnliches. Derartige Temperatursensoren 140 sind grundsätzlich bekannt.

Weiterhin kann das Netzwerk 126 beispielsweise einen oder mehrere Blutdrucksensoren 142, Analytsensoren 144 oder andere Arten von Sensoren umfassen. Die Sensoren sind in Figur 2 allgemein mit der Bezugsziffer 146 bezeichnet.

Alternativ oder zusätzlich zu Sensoren 146 können die Netzwerkknoten 132 andere Arten medizinischer Funktionen umfassen, beispielsweise medizinische einsetzbare Aktoren 148. Beispielsweise kann ein Netzwerkknoten 132 mit einer Medikationsvorrichtung 150 in Form einer Insulinpumpe 152 vorgesehen sein. Auch andere Arten von Medikationsvorrichtungen 150 können alternativ oder zusätzlich vorgesehen sein, welche auch allgemein als "Drug Delivery"-Systeme 154 bezeichnet werden können.

In dem Ausführungsbeispiel des Netzwerkes 126 gemäß Figur 2 umfasst dieses Netzwerk 126 eine Anzeigevorrichtung 156. Diese ist im vorliegenden Ausführungsbeispiel in einer Armbanduhr 158 aufgenommen, welche entsprechend ausgestaltet ist, um in das Netzwerk 126 integriert zu werden. Beispielsweise kann eine entsprechende programmtechnische Einrichtung dieser Armbanduhr 158 erfolgen, und die Armbanduhr 158 kann ebenfalls als Netzwerkknoten 132 über Elektroden 116 und Sender 112 und/oder Empfänger 114 verfügen, sowie ggf. über weitere erforderliche Einrichtungen, wie beispielsweise mindestens einen Signalgenerator 122 und/oder mindestens einen Verstärker 123.

Die Armbanduhr 158 kann somit als visuelle Schnittstelle zwischen dem Patienten 128 und dem Netzwerk 126 dienen. Darüber hinaus kann die Armbanduhr 158 auch als Netzwerkknoten 132 mit Eingabefunktionen verwendet werden, beispielsweise um es dem Patienten 128 zu ermöglichen, Befehle einzugeben, das Netzwerk 126 zu steuern und/oder Informationen aus dem Netzwerk 126 abzufragen.

Optional umfasst das Netzwerk 126 bei dem in Figur 2 dargestellten Ausführungsbeispiel weitere Netzwerkknoten 132 mit Anzeigefunktion und/oder Ein- und Ausgabemitteln. So können beispielsweise ein oder mehrere Handgeräte 160 umfasst sein, welche ebenfalls als Netzwerkknoten 132 eingebunden sein können. Diese Handgeräte 160 können ein oder mehrere Mobiltelefone 162, tragbare Computer 164 (z.B. so genannte PDAs, Personal Digital Assistants) oder tragbare Messgeräte 166, beispielsweise Blutzuckermessgeräte, umfassen. Diese können beispielsweise über eine Hand 168 des Patienten 128 in das Netzwerk 126 eingebunden sein, um beispielsweise Kalibrationsdaten 170 oder Ähnliches mit übrigen Netzwerkknoten 132 auszutauschen. Auch Steuerbefehle, Messdaten oder Ähnliches können auf diese Weise ausgetauscht werden.

Wie in Figur 2 ebenfalls angedeutet, kann das Netzwerk 126 auch in ein medizinisches System 130 eingebunden sein, beispielsweise ein so genanntes Healthcare-System. Das Netzwerk 126 kann sich auch automatisch in ein oder mehrere Healthcare-Systeme einschalten und diese unterstützen, beispielsweise im Fall einer Notfalldiagnostik, beispielsweise bei einem Notarzt-Einsatz, in einem Krankenwagen, bei einer Anästhesie, während einer Operation oder in ähnlichen Situationen. Ein Vorteil der Verwendung des Netzwerks 126 besteht in diesem Fall darin, dass beispielsweise die Sensoren 146 und/oder andere Komponenten des Netzwerks 126 nicht erst gelegt werden müssen, sondern bereits zumindest teilweise am Patienten vorhanden sind. Dieses medizinische System 130 kann beispielsweise mit dem Netzwerk 126 über eine Datenverbindung 172 Messdaten, Informationen, Steuerbefehle oder Ähnliches austauschen. Beispielsweise können zu diesem Zweck Fernfeldkommunikationen genutzt werden, beispielsweise über ein Mobiltelefon 162 des Netzwerkes 126. Das medizinische System 130 kann beispielsweise einen oder mehrere Computer 174 umfassen und/oder Computernetzwerke, wie in Figur 2 ebenfalls dargestellt. Das medizinische System 130 kann weiterhin ein oder mehrere Kommunikationsvorrichtungen 175 umfassen, welche beispielsweise auch Bestandteil des Computers 174 und/oder des Computernetzwerkes sein können. Mittels dieser mindestens einen Komnunikationsvorrichtung 175 kann beispielsweise die Datenverbindung 172 zum Netzwerk 126 aufgebaut und aufrechterhalten werden.

Die Ausgestaltung des Netzwerkes 126 in dem Ausführungsbeispiel gemäß Figur 2 ist lediglich exemplarisch zu verstehen. Das Netzwerk 126 kann auch auf erheblich abweichende Art ausgestaltet sein, mit einer abweichenden Anordnung der Netzwerkknoten 132 und/oder abweichenden Funktionen. So können beispielsweise in dem gezeigten Ausführungsbeispiel in einem menschlichen oder tierischen Körper 110 ein oder mehrere interstitielle Glucosesensoren teilimplantiert oder vollständig implantiert sein. Ebenso sind weitere Analytsensoren 144 alternativ oder zusätzlich implantierbar. Extrakorporal sind weitere physikalische Sensoren 146 einsetzbar, wie beispielsweise der beschrieben Blutdrucksensor 142, ein Oxymeter, ein Pulsmesser oder Ähnliches.

Für einen Körperstatus können insbesondere bei Patienten 128 mit kritischem Gesamtzustand, alternativ oder zusätzlich, weitere physikalische und/oder chemische Parameter durch die Sensoren 146 erfasst werden. So können beispielsweise Lactat, CO₂, HB, HB-O₂, Nierenparameter (insbesondere in Zusammenhand mit Multiorganversagen), Harnfunktionen oder Kombinationen der genannten Parameter oder anderer Parameter gemessen werden. Darüber hinaus können die Sensoren 146 alternativ oder zusätzlich beispielsweise Bewegungsmelder umfassen. An Aktoren 148 kommen, neben den in einer Medikationsvorrichtung 150 einsetzbaren Aktoren (beispielsweise Dosierungsaktoren), alternativ oder zusätzlich auch andere Arten von Aktoren in Betracht, beispielsweise Ventile, beispielsweise zur Harnsteuerung.

Weiterhin können Aktoren 148 beispielsweise in der Insulinpumpe 152 und/oder in anderen Arten von Medikationsvorrichtung 150 eingesetzt werden. Die Insulinpumpe 152 kann beispielsweise extrakorporal angeordnet sein, beispielsweise mit einem implantierbaren Katheder. Alternativ oder zusätzlich können andere Arten von "Drug Delivery"- Systemen 154 eingesetzt werden, welche optional ebenfalls einen oder mehrere Aktoren 146 umfassen können.

Die Armbanduhr 158 mit der Anzeigevorrichtung 156 fungiert hier vorzugsweise als permanentes Display, beispielsweise für eine Statusanzeige oder eine Alarmanzeige. Die Anzeigevorrichtung 156 kann beispielsweise eine optische und/oder eine akustische Ausgabe von Informationen ermöglichen. Alternativ oder zusätzlich können, insbesondere sporadisch, auch weitere Geräte in das Netzwerk 126 eingebunden werden, wie in Figur 2 durch die Handgeräte 160 angedeutet. Neben dem Mobiltelefon und dem tragbaren Computer 164 kommen insbesondere auch tragbare Messgeräte 116 in Betracht, beispielsweise Blutzuckermessgeräte, Blutdruckmessgeräte oder Ähnliches. Allgemein können diese Handgeräte 160 in die Hand 168 des Patienten genommen werden und somit zumindest temporär als Teil des Netzwerkes 126 eingebunden werden. Für die "near field intrabody communication" entsprechend geeignete Elektroden 116, welche in Figur 2 nicht dargestellt sind, können beispielsweise an diesen Handgeräten 160 ausgebildet sein. Derartige temporäre Netzwerkknoten 132 mit Handgeräten 160 können beispielsweise der Kontrolle, Initialisierung und/oder Kalibration weiterer Komponenten des Netzwerkes 126 dienen. Allgemein sei darauf hingewiesen, dass der Begriff des "Handgerätes" derartige Geräte jedoch nicht notwendigerweise auf tragbare Geräte beschränkt. Allgemein handelt es sich dabei um Geräte, welche zwar vorzugsweise tragbar sind, welche jedoch grundsätzlich auch stationär ausgebildet sein können und einen Kontakt mit einer Hand 168 des Patienten herstellen können.

In dem dargestellten Ausführungsbeispiel gemäß Figur 2 kann beispielsweise ein so genanntes Spot-Blutglucose-Messgerät als tragbares Messgerät 166 eingesetzt werden. Dieses kann beispielsweise bei Handkontakt einen zeitnah gemessenen Blutglucosewert direkt an das kontinuierliche Messsystem des Glucosesensors 136 mit dem implantierbaren Sensor 138, welches Glucose im Interstitium des Patienten 128 misst, als Basis für eine Kalibration übertragen. Diese ist beispielsweise Voraussetzung für eine künstliche Bauchspeicheldrüse ("artificial pancreas").

Ferner wäre es denkbar, dass Vollblut-Messsysteme als Glucosesensor 136 und/oder als tragbares Messgerät 166 und/oder in weiteren Netzwerkknoten 132 eingesetzt werden. Diese können beispielsweise mit Vorrichtungen zur minimalinvasiven Blutgewinnung und/oder unmittelbaren Messung ausgestattet sein. Von derartigen Messsystemen kann dann beispielsweise eine Blutgewinnungszeitpunkt und/oder ein Messzeitpunkt an diverse Netzwerkknoten 132 übertragen werden.

Neben der Einbindung in das Netzwerk 126 können einer oder mehrere der Netzwerkknoten 132 auch eingerichtet sein, um eine Kommunikation außerhalb des Netzwerkes 126 durchzuführen, beispielsweise über die Datenverbindung 172. Neben einer drahtgebundenen Datenübertragung kommen auch drahtlose Übertragungstechniken in Betracht, beispielsweise sämtliche bekannten Übertragungstechniken, beispielsweise die eingangs genannten Arten der Übertragungstechniken. Insbesondere kann hierbei dann auch eine Fernfeldübertragung genutzt werden. So können beispielsweise Netzwerkknoten 132, welche mit der Hand 168 verbunden sind, derartige Übertragungsfunktionen übernehmen. Beispielsweise können die Handgeräte 160 eingerichtet sein, insbesondere das Mobiltelefon 162, um beispielsweise eine bidirektionale Verbindung im Fernfeld herzustellen. Auch die Armbanduhr 158 wäre, alternativ oder zusätzlich, ein Beispiel eines hierzu geeigneten Elementes.

Weiterhin sei darauf hingewiesen, dass in Figur 2 als Beispiel eine sternförmige Kommunikationsstruktur des Netzwerks 126 dargestellt ist. Dabei übernimmt beispielsweise der Glucosesensor 136, welcher beispielsweise als Glucose-Patch mit einem implantierbaren Sensor 138 ausgestaltet sein kann, die Rolle des "Masters". Prinzipiell können jedoch andere Netzwerkknoten 132 diese Rolle alternativ oder zusätzlich übernehmen. Die Übernahme der Rolle des Masters kann dabei von den jeweiligen Komponenten dauerhaft oder auch temporär übernommen werden. Weiterhin können auch andere Kommunikationsstrukturen als die genannte sternförmige Struktur eingesetzt werden.

Der Master-Netzwerkknoten 134 kann beispielsweise den Kommunikationsverkehr koordinieren und kann darüber hinaus gegebenenfalls die Aufgabe haben, multivariante Parameter nach definierten Parametern zu verknüpfen und gegebenenfalls Anweisungen an andere Netzwerkknoten 132, beispielsweise die Aktoren 148, zu generieren. Auch selbstlernende Softwarestrukturen sind denkbar. Prinzipiell können auch andere Netzwerkknoten 132 diese Aufgabe übernehmen. Beispielsweise sind Strukturen denkbar, in welchen sich das Netzwerk 126 selbst organisiert und der jeweils am besten geeignete Netzwerkknoten 132 die Aufgabe des Master-Netzwerkknotens 134 übernimmt, beispielsweise dauerhaft oder temporär.

Prinzipiell kann die Kommunikation 126 gemäß bekannter asynchroner Netze erfolgen. Jeder der Netzwerkknoten 132 kann beispielsweise eine spezifische Adresse aufweisen, über die das Ansprechen dieses Netzwerkknotens 132 erfolgen kann. Eine Datenübertragung kann paketorientiert erfolgen. Dabei kann beispielsweise ein Telegramm in Pakete zerlegt und über Paketnummern im jeweiligen Empfänger in eine zeitliche Reihenfolge gebracht werden. Bei einer Störung einzelner Pakete können diese Pakete solange wiederholt gesendet werden, bis ein oder mehrere Kontrollmechanismen, beispielsweise ein so genannter CRC-Check, die Übertragung für korrekt halten.

Da in der Regel davon auszugehen sein wird, dass die übertragene Energie sehr niedrig ist und dass das Stör-zu-Signal-Verhältnis vergleichsweise schlecht ist, können gegebenenfalls auch neuartige Protokolle mit hoher Redundanz entwickelt werden. Dies ist möglich, da die Informationsdichte zwischen den Netzwerkknoten 132 in der Regel vergleichsweise gering sein wird, so dass eine hohe Bandbreite für eine erhöhte Redundanz und/oder für eine geringe Latenzzeit verwendet werden kann.

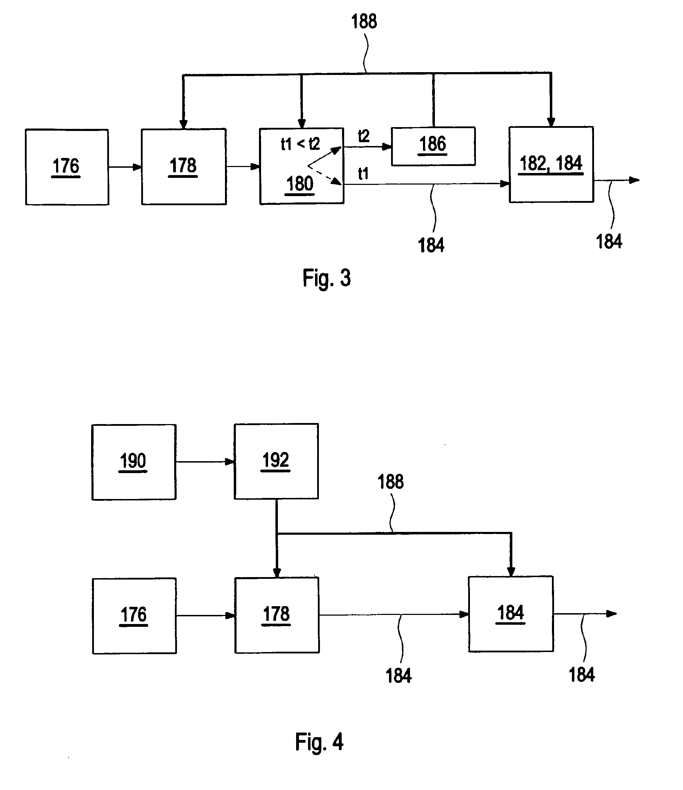

Eine Problematik in typischen medizinischen Netzwerken, wie beispielsweise den in Figur 2 dargestellten Netzwerken 126, stellt üblicherweise die Energieversorgung des gesamten Netzwerkes 126 und/oder einzelner Netzwerkknoten 132 des Netzwerkes 126 dar. In den Figuren 3 und 4 sind verschiedene schematische Ausführungsbeispiele einer möglichen Energieversorgung dargestellt, welche in einem Netzwerkknoten 132, in mehreren Netzwerkknoten 132 oder in allen Netzwerkknoten 132 eingesetzt werden können. Als Beispiel ist hier ein "energy harvesting", also eine Energiegewinnung, im Umfeld des Glucosesensors 136 gezeigt. Grundsätzlich lassen sich die Prinzipien jedoch auch auf andere Arten von Netzwerkknoten 132 und/oder auf andere Arten von Funktionen anwenden. Dabei zeigt Figur 3 eine Prinzipskizze einer Energiegewinnung, bei welcher eine Signalgewinnung eines Sensors 146 und eine Energiegewinnung für den Betrieb des Netzwerkknotens 132 und/oder einzelner Komponenten des Netzwerkknotens 132 und/oder anderer Komponenten des Netzwerkens 126 aus derselben Quelle erfolgt. Figur 4 zeigt hingegen ein Ausführungsbeispiel bei welchem die Energiegewinnung aus einer separaten Quelle erfolgt.

Bei der Energiegewinnung aus derselben Quelle gemäß Figur 3 wird ein biochemisches System 176 eingesetzt. Dieses kann beispielsweise ein biochemisches Redoxsystem sein, welches Ladung und/oder Strom generiert. Beispielsweise kann dies ein üblicherweise in Blutglucosesensoren eingesetztes elektrochemisches System sein, welches auf einer Oxidation der Blutglucose basiert und welches gegebenenfalls Enzyme und/oder Hilfsstoffe verwendet.

Hintergrund für den in Figur 3 dargestellten Energiegewinnungsgedanken ist, dass eine derartige Struktur eines biochemischen Systems 176 für die Messung, d.h. für den eigentlichen Messtakt des Sensors 146, nur vergleichsweise wenig Energie benötigt. Beispielsweise wird typischerweise für die Messung lediglich 1/1000 der ständig fließenden Ladung benötigt. Der Rest muss in der Regel abgeführt und in Wärme umgewandelt werden, damit sich die Ladung nicht am Messort des Sensors 146 anreichert. Dieser in der Regel abgeführte Anteil kann jedoch, wie in Figur 3 angedeutet, auch zur Energiegewinnung gesammelt werden.

So umfasst das Ausführungsbeispiel gemäß Figur 3 beispielsweise, angeschlossen an das biochemische System 176, optional einen Wandler 178, beispielsweise einen Wandler mit low-voltage-start. Dieser Wandler kann für die Energiegewinnung genutzt werden. Angeschlossen an diesen Wandler 178 wiederum ist ein Schalter 180, welcher zwischen zwei Modi umschalten kann: In einem Sensormodus 182 wird mindestens eine Messgröße des Sensors 146 erfasst, beispielsweise ein Strom und/oder eine Spannung. Diese mindestens eine Messgröße kann beispielsweise als Signal übertragen werden, was in Figur 3 durch die Bezugsziffer 184 angedeutet ist. Verschiedene Ausgestaltungen sind denkbar. Diese Signalübertragung 184 kann beispielsweise an weitere Komponenten des Netzwerkknoten 132 und/oder an externe Komponenten erfolgen.

In einem weiteren Modus, der in Figur 3 symbolisch als Energiegewinnungsmodus 186 bezeichnet ist, kann hingegen, wie oben beschrieben, die überschüssige Ladung, der überschüssige Strom oder die nicht verwendete Spannung für die Energiegewinnung genutzt werden. Dies erfolgt in diesem Beispiel, aufgrund des Umschaltens zwischen den Modi derart, dass die Energieentnahme in keinem Fall den Messwert beeinflusst, beispielsweise durch Verarmung der Glukose im Bereich des Messorts. Auf diese Weise kann beispielsweise Energie für den Sensor 146, den Netzwerkknoten 132 und/oder weitere Komponenten des Netzwerkes 126 erzeugt und bereitgestellt werden. In Figur 3 ist dies symbolisch durch den Bereitstellungspfeil 188 angedeutet, welcher zeigt, dass beispielsweise der Wandler 178 und/oder der Schalter 180 und/oder die Signalübertragung 184 auf diese Weise mit elektrischer Energie versorgt werden können.

Es wird darauf hingewiesen, dass die Bezeichnung der Bezugsziffer 186 für den Energiegewinnungsmodus in Figur 3 lediglich symbolisch zu verstehen ist. Der in Figur 3 mit der Bezugsziffer 186 bezeichnete Block kann auch apparative Elemente umfassen, welche mit dem Energiegewinnungsmodus in Verbindung stehen. So kann der Energiegewinnungsmodus 186 beispielsweise auch allgemein eine Umwandlung von Energie und/oder mindestens einen in Figur 3 nicht näher bezeichneten Energiespeicher umfassen.

Das Umschalten zwischen den beiden beschriebenen Modi kann beispielsweise, wie in Figur 3, durch die Zeiten t₁ und t₂ zeitlich gesteuert erfolgen. Auch andere Umschaltverfahren sind denkbar, d.h. neben zeitlich gesteuerten, beispielsweise getakteten, Verfahren sind beispielsweise auch zeitlich flexible Verfahren, welche beispielsweise konkret auf eine Messanfrage reagieren können, denkbar. Insgesamt kann auf die beschriebene Weise gemäß Figur 3 bei einem praktisch realisierbaren Sensor 146 beispielsweise ca. 1 µWs Energie erzeugt werden. Auf der Elektronikseite sind dementsprechend aufgrund der knappen Energieressourcen energiesparende Anwendungen bevorzugt.

In Figur 4 ist hingegen ein Konzept gezeigt, bei welchem die Energiegewinnung aus einer separaten Quelle erfolgt. Beispielsweise kann wiederum ein biochemisches System 176 vorgesehen sein, beispielsweise in einem Sensor 146. Auch andere Arten von Sensoren 146 und/oder Aktoren 148 sind jedoch grundsätzlich einsetzbar. Weiterhin ist wiederum ein Messwert-Wandler 178 vorgesehen, sowie eine entsprechende Signalübertragung 184.

Im Unterschied zur Ausführungsform gemäß Figur 3 erfolgt jedoch in Figur 4 eine separate Energiegewinnung. Dementsprechend ist eine Energiegewinnungsvorrichtung 190 vorgesehen, welche vorzugsweise dem Körper 110 und/oder einer Umgebung des Körpers 110 eine Energie entzieht. Beispielsweise kann eine Bewegungsenergie mittels piezo-elektrischer Elemente generiert werden, eine thermische Energie in Form von Temperaturunterschieden oder Ähnliches. Diese gewonnene Energie kann beispielsweise in einem Energiespeicher 192 zwischengespeichert werden und dann an weitere Systemkomponenten bereitgestellt werden. Die Bereitstellung ist wiederum mit der Bezugsziffer 188 bezeichnet. Exemplarisch werden in dem in Figur 4 dargestellten Ausführungsbeispiel der Wandler 178 und die Signalübertragung 184 mit elektrischer Energie versorgt.

Das Konzept der separaten Energiegewinnung gemäß dem Ausführungsbeispiel in Figur 4 weist gegenüber der Energiegewinnung gemäß Figur 3 weiterhin den Vorteil auf, dass eine parallele Energiegewinnung in der Regel zu einer höheren und unabhängigeren Energieentnahme führen kann. Bei dem Aufbau gemäß Figur 3 hingegen kann bei abnehmendem Energieverbrauch der Verarbeitungselektronik ein Rauschproblem auftreten, welches jedoch ebenfalls durch entsprechende Vorkehrungen, beispielsweise eine Integration des Signals, verringert werden kann. Die Parallel-Gewinnung von Energie gemäß Figur 4 erfordert in der Regel keine derartigen zusätzlichen Vorkehrungen.

Es sei darauf hingewiesen, dass das Netzwerk 126 alternativ oder zusätzlich zu den Konzepten der Energiegewinnung in den Figuren 3 und/oder 4, auch einen oder mehrere weitere Energiespeicher 192 umfassen kann. Beispielsweise können ein oder mehrere Batterien, Akkumulatoren, Superkondensatoren oder Ähnliches als Energiespeicher vorgesehen sein, wobei wiederaufladbare und/oder nicht-wiederaufladbare Energiespeicher vorgesehen sein können.

### Bezugszeichenliste

| | |
|---|---|
| 110 | Körper |
| 112 | Sender |
| 114 | Empfänger |
| 116 | Elektroden |
| 118 | Hautoberfläche |
| 120 | Energiequelle |
| 122 | Signalgenerator |
| 123 | Verstärker |
| 124 | Elektrisches Feld |
| 126 | Netzwerk |
| 128 | Patient |
| 130 | Medizinisches System |
| 132 | Netzwerkknoten |
| 134 | Master-Netzwerkknoten |
| 136 | Glucosesensor |
| 138 | Implantierbarer Sensor |
| 140 | Temperatursensor |
| 142 | Blutdrucksensor |
| 144 | Analytsensor |
| 146 | Sensor |
| 148 | Aktor |
| 150 | Medikationsvorrichtung |
| 152 | Insulinpumpe |
| 154 | Drug Delivery System |
| 156 | Anzeigevorrichtung |
| 158 | Armbanduhr |
| 160 | Handgerät |
| 162 | Mobiltelefon |
| 164 | Tragbarer Computer |
| 166 | Tragbares Messgerät |
| 168 | Hand |
| 170 | Kalibrationsdaten |
| 172 | Datenverbindung |
| 174 | Computer |
| 175 | Kommunikationsvorrichtung |
| 176 | Biochemisches System |
| 178 | Wandler |
| 180 | Schalter |
| 182 | Sensormodus |
| 184 | Signalübertragung |
| 186 | Energiegewinnungsmodus |
| 188 | Bereitstellungsenergie |
| 190 | Energiegewinnungsvorrichtung |
| 192 | Energiespeicher |

## Patentansprüche

1. Netzwerk (126) zur Überwachung von Körperfunktionen eines Patienten (128), umfassend mindestens zwei verschiedene, mit einem Körper (110) des Patienten (128) verbindbare Netzwerkknoten (132), wobei mindestens zwei der Netzwerkknoten (132) jeweils mindestens eine medizinische Funktion aufweisen, insbesondere eine diagnostische Funktion und/oder eine Medikationsfunktion, wobei die Netzwerkknoten (132) eingerichtet sind, um über den Körper (110) des Patienten (128) unmittelbar miteinander zu kommunizieren und Daten und/oder Befehle auszutauschen, **dadurch gekennzeichnet, dass** mindestens zwei der Netzwerkknoten (132) eingerichtet sind, um eine Steuerung des Netzwerks (126) zu übernehmen.

2. Netzwerk (126) nach dem vorhergehenden Anspruch, wobei mindestens einer der Netzwerkknoten (132) mindestens einen der folgenden Sensoren (146) umfasst: einen Sensor (144) zur Erfassung mindestens eines Analyten in einer Körperflüssigkeit, insbesondere Glukose, Lactat, CO₂, HB, HB-O₂; einen Sensor (146) zur Erfassung mindestens einer Körperfunktion, insbesondere einer Nierenfunktion; einen Blutdrucksensor (142); ein Oxymeter; einen Pulsmesser; einen Bewegungsmelder; einen Temperatursensor (140).

3. Netzwerk (126) nach einem der vorhergehenden Ansprüche, wobei mindestens einer der Netzwerkknoten (132) mindestens einen ganz oder teilweise in den Körper (110) des Patienten (128) implantierbaren Sensor (138, 146) umfasst, wobei der implantierbare Sensor (138, 146) eingerichtet ist, um mindestens eine Messgröße in einem Körpergewebe und/oder einer Körperflüssigkeit zu erfassen, insbesondere eine Konzentration mindestens eines Analyten.

4. Netzwerk (126) nach einem der vorhergehenden Ansprüche, wobei mindestens einer der Netzwerkknoten (132) mindestens einen Aktor (148) umfasst, insbesondere einen oder mehrere der folgenden Aktoren (148): einen Aktor zur Beeinflussung mindestens einer Körperfunktion, insbesondere einen elektrischen Aktor und/oder einen mechanischen Aktor; ein Ventil, insbesondere ein Ventil für eine Harnsteuerung; einen Medikationsaktor, insbesondere eine Medikationspumpe (152).

5. Netzwerk (126) nach einem der vorhergehenden Ansprüche, wobei mindestens einer der Netzwerkknoten (132) mindestens einen Sensor (146) zur Erfassung mindestens einer Messgröße umfasst und wobei mindestens ein anderer der Netzwerkknoten (132) mindestens eine therapeutische Vorrichtung umfasst, insbesondere eine Medikationsvorrichtung (150), wobei das Netzwerk (126) eingerichtet ist, um über den Körper (110) des Patienten (128) die therapeutische Vorrichtung entsprechend der Messgröße zu steuern und/oder zu regeln.

6. Netzwerk (126) nach einem der vorhergehenden Ansprüche, wobei alle der Netzwerkknoten (132) eingerichtet sind, um eine Steuerung des Netzwerks (126) zu übernehmen.

7. Netzwerk (126) nach einem der vorhergehenden Ansprüche, wobei die Netzwerkknoten (132) eingerichtet sind, um über eine asynchrone Datenübertragung zu kommunizieren.

8. Netzwerk (126) nach einem der vorhergehenden Ansprüche, wobei mindestens einer der Netzwerkknoten (132) eine Anzeigevorrichtung (156) umfasst, insbesondere eine an einem Handgelenk des Patienten (128) tragbare Anzeigevorrichtung (156), insbesondere eine in eine Armbanduhr (158) integrierte Anzeigevorrichtung (156).

9. Netzwerk (126) nach einem der vorhergehenden Ansprüche, wobei mindestens einer der Netzwerkknoten (132) eingerichtet ist, um eine Failsafe-Funktion durchzuführen und um bei Erkennung eines Fehlerzustandes mindestens eine Fehlerroutine durchzuführen.

10. Netzwerk (126) nach einem der vorhergehenden Ansprüche, wobei mindestens einer der Netzwerkknoten (132) eingerichtet ist, um einen Alarm an den Patienten (128) auszugeben, insbesondere im Fall einer Fehlfunktion des Netzwerks (126) und/oder im Fall eines Auftretens abnormaler Körperfunktionen.

11. Netzwerk (126) nach einem der vorhergehenden Ansprüche, wobei mindestens einer der Netzwerkknoten (132) zusätzlich für eine Kommunikation außerhalb des Körpers (110) eingerichtet ist, insbesondere eine Fernfeldkommunikation, insbesondere zum Upload und/oder Download von Daten.

12. Netzwerk (126) nach einem der vorhergehenden Ansprüche, wobei mindestens einer der Netzwerkknoten (132) eingerichtet ist, um dem Körper (110) und/oder einer Umgebung des Körpers (110) Energie zu entziehen und diese für eine eigene Energieversorgung und/oder eine Energieversorgung anderer Netzwerkknoten (132) zu nutzen.

13. Netzwerk (126) nach dem vorhergehenden Anspruch, wobei der Netzwerkknoten (132) mindestens einen elektrochemischen Sensor (146) umfasst, wobei der elektrochemische Sensor (146) eingerichtet ist, um in einem Sensormodus mindestens eine Messgröße zu erfassen und wobei der elektrochemische Sensor (146) weiterhin eingerichtet ist, um in einem Energiegewinnungsmodus elektrochemisch Energie zu gewinnen.

14. Netzwerk (126) nach einem der vorhergehenden Ansprüche, weiterhin umfassend mindestens ein in das Netzwerk (126) integrierbares und aus dem Netzwerk (126) abkoppelbares tragbares Handgerät (160) mit mindestens einer Anzeigefunktion, insbesondere ein medizinisches Messgerät (166) und/oder ein Mobiltelefon (162), wobei das Netzwerk (126) eingerichtet ist, um bei einem Kontakt des Handgeräts (160) mit dem Körper (110) des Patienten (128), insbesondere einer Hand (168) des Patienten (128), das Handgerät (160) automatisch in das Netzwerk (126) einzubinden.

15. Netzwerkknoten (132) zum Einsatz in einem Netzwerk (126) nach einem der vorhergehenden Ansprüche, umfassend mindestens eine medizinische Funktion, insbesondere eine diagnostische Funktion und/oder eine Medikationsfunktion, wobei der Netzwerkknoten (132) mindestens eine mit dem Körper (110) des Patienten (128) verbindbare Kommunikationseinheit umfasst und eingerichtet ist, um über den Körper (110) des Patienten (128) unmittelbar mit anderen Netzwerkknoten (132) des Netzwerks (126) zu kommunizieren und Daten und/oder Befehle auszutauschen, **dadurch gekennzeichnet, dass** der Netzwerkknoten (132) derart eingerichtet ist, dass mindestens zwei der Netzwerkknoten (132) eine Steuerung des Netzwerks (126) übernehmen können.

## Claims

1. Network (126) for monitoring bodily functions of a patient (128), comprising at least two distinct network nodes (132) that can be connected to a body (110) of the patient (128), wherein at least two of the network nodes (132) respectively have at least one medical function, more particularly a diagnostic function and/or a medication function, wherein the network nodes (132) are designed to communicate directly with one another via the body (110) of the patient (128) and to interchange data and/or commands, **characterized in that** at least two of the network nodes (132) are designed to take over a control of the network (126).

2. Network (126) according to the preceding claim, wherein at least one of the network nodes (132) comprises at least one of the following sensors (146): a sensor (144) for registering at least one analyte in a bodily fluid, more particularly glucose, lactate, CO₂, Hb, Hb-O₂; a sensor (146) for registering at least one bodily function, more particularly a kidney function; a blood-pressure sensor (142); an oximeter; a heart-rate monitor; a motion detector; a temperature sensor (140).

3. Network (126) according to one of the preceding claims, wherein at least one of the network nodes (132) comprises at least one sensor (138, 146) that can wholly or partly be implanted into the body (110) of the patient (128), wherein the implantable sensor (138, 146) is designed to register at least one measurement variable in a body tissue and/or a bodily fluid, more particularly a concentration of at least one analyte.

4. Network (126) according to one of the preceding claims, wherein at least one of the network nodes (132) comprises at least one actuator (148), more particularly one or more of the following actuators (148): an actuator for influencing at least one bodily function, more particularly an electrical actuator and/or a mechanical actuator; a valve, more particularly a valve for urinary control; a medication actuator, more particularly a medication pump (152).

5. Network (126) according to one of the preceding claims, wherein at least one of the network nodes (132) comprises at least one sensor (146) for registering at least one measurement variable and wherein at least one other network node (132) comprises at least one therapeutic device, more particularly a medication device (150), wherein the network (126) is designed to control and/or regulate the therapeutic device according to the measurement variable via the body (110) of the patient (128).

6. Network (126) according to one of the preceding claims, wherein all network nodes (132) are designed to take over a control of the network (126).

7. Network (126) according to one of the preceding claims, wherein the network nodes (132) are designed to communicate over asynchronous data transmission.

8. Network (126) according to one of the preceding claims, wherein at least one of the network nodes (132) comprises an indication device (156), more particularly an indication device (156) that can be worn on a wrist of the patient (128), more particularly an indication device (156) integrated into a wrist watch (158).

9. Network (126) according to one of the preceding claims, wherein at least one of the network nodes (132) is designed to carry out a failsafe function and to carry out at least one error routine if an error state is identified.

10. Network (126) according to one of the preceding claims, wherein at least one of the network nodes (132) is designed to emit an alarm to the patient (128), particularly if there is a malfunction of the network (126) and/or if abnormal bodily functions occur.

11. Network (126) according to one of the preceding claims, wherein at least one of the network nodes (132) is designed for communication outside of the body (110), more particularly for far-field communication, more particularly for uploading and/or downloading data.

12. Network (126) according to one of the preceding claims, wherein at least one of the network nodes (132) is designed to extract energy from the body (110) and/or from surroundings of the body (110) and to use this energy to supply said network node and/or other network nodes (132) with energy.

13. Network (126) according to the preceding claim, wherein the network node (132) comprises at least one electrochemical sensor (146), wherein the electrochemical sensor (146) is designed to register at least one measurement variable in a sensor mode and wherein the electrochemical sensor (146) is furthermore designed to extract energy by electrochemical means in an energy-extraction mode.

14. Network (126) according to one of the preceding claims, furthermore comprising at least one portable hand-held instrument (160), more particularly a medical measurement instrument (166) and/or a cellular telephone (162), which hand-held instrument has at least one indication function and can be integrated into the network (126) and can be decoupled from the network (126), wherein the network (126) is designed to link automatically the hand-held instrument (160) into the network (126) when the hand-held instrument (160) makes contact with the body (110) of the patient (128), more particularly a hand (168) of the patient (128).

15. Network node (132) for use in a network (126) according to one of the preceding claims, comprising at least one medical function, more particularly a diagnostic function and/or a medication function, wherein the network node (132) comprises at least one communication unit, which can be connected to the body (110) of the patient (128), and is designed to communicate directly with other network nodes (132) of the network (126) via the body (110) of the patient (128) and to interchange data and/or commands, **characterized in that** the network node (132) is designed in such a way that at least two of the network nodes (132) can take over a control of the network (126).

## Revendications

1. Réseau (126) de surveillance des fonctions corporelles d'un patient (128), comprenant au moins deux noeuds de réseau (132) différents pouvant être reliés avec le corps (110) du patient (128), au moins deux des noeuds de réseau (132) présentant respectivement au moins une fonction médicale, notamment une fonction de diagnostic et/ou une fonction d'administration de médicament, les noeuds de réseau (132) étant configurés pour communiquer directement entre eux par le biais du corps (110) du patient (128) et pour échanger des données et/ou des instructions, **caractérisé en ce qu'**au moins deux des noeuds de réseau (132) sont configurés pour prendre en charge une commande du réseau (126).

2. Réseau (126) selon la revendication précédente, avec lequel au moins l'un des noeuds de réseau (132) comprend au moins l'un des capteurs (146) suivants : un capteur (144) pour détecter au moins une substance à analyser dans un liquide corporel, notamment du glucose, du lactate, du CO₂, du HB, du HB-O₂ ; un capteur (146) pour détecter au moins une fonction corporelle, notamment une fonction rénale ; un capteur de pression sanguine (142) ; un oxymètre ; un pulsomètre ; un détecteur de mouvement ; une sonde de température (140).

3. Réseau (126) selon l'une des revendications précédentes, avec lequel au moins l'un des noeuds de réseau (132) comprend au moins un capteur (138, 146) implantable totalement ou partiellement dans le corps (110) du patient (128), le capteur implantable (138, 146) étant configuré pour détecter au moins une grandeur mesurée dans un tissu corporel et/ou un liquide corporel, notamment une concentration d'au moins une substance à analyser.

4. Réseau (126) selon l'une des revendications précédentes, avec lequel au moins l'un des noeuds de réseau (132) comprend au moins un actionneur (148), notamment un ou plusieurs des actionneurs (148) suivants : un actionneur pour influencer au moins une fonction corporelle, notamment un actionneur électrique et/ou un actionneur mécanique ; une vanne, notamment une vanne pour une commande d'urine ; un actionneur d'administration de médicament, notamment une pompe d'administration de médicament (152).

5. Réseau (126) selon l'une des revendications précédentes, avec lequel au moins l'un des noeuds de réseau (132) comprend au moins un capteur (146) pour détecter au moins une grandeur mesurée et avec lequel au moins un autre des noeuds de réseau (132) comprend au moins un dispositif thérapeutique, notamment un dispositif d'administration de médicament (150), le réseau (126) étant configuré pour commander et/ou réguler le dispositif thérapeutique par le biais du corps (110) du patient (128) en fonction de la grandeur mesurée.

6. Réseau (126) selon l'une des revendications précédentes, avec lequel tous les noeuds de réseau (132) sont configurés pour prendre en charge une commande du réseau (126).

7. Réseau (126) selon l'une des revendications précédentes, avec lequel les noeuds de réseau (132) sont configurés pour communiquer par le biais d'une transmission de données asynchrone.

8. Réseau (126) selon l'une des revendications précédentes, avec lequel au moins l'un des noeuds de réseau (132) présente un dispositif d'affichage (156), notamment un dispositif d'affichage (156) pouvant être porté à un poignet du patient (128), notamment un dispositif d'affichage (156) intégré dans une montre-bracelet (158).

9. Réseau (126) selon l'une des revendications précédentes, avec lequel au moins l'un des noeuds de réseau (132) est configuré pour exécuter une fonction de sécurité intégrée et pour exécuter au moins une routine de défaut en cas de détection d'une situation de défaut.

10. Réseau (126) selon l'une des revendications précédentes, avec lequel au moins l'un des noeuds de réseau (132) est configuré pour délivrer une alarme au patient (128), notamment dans le cas d'un dysfonctionnement du réseau (126) et/ou dans le cas où il se produit des fonctions corporelles anormales.

11. Réseau (126) selon l'une des revendications précédentes, avec lequel au moins l'un des noeuds de réseau (132) est en plus configuré pour une communication à l'extérieur du corps (110), notamment une communication par champ distant, notamment pour le téléversement et/ou le téléchargement de données.

12. Réseau (126) selon l'une des revendications précédentes, avec lequel au moins l'un des noeuds de réseau (132) est configuré pour prélever de l'énergie du corps (110) et/ou de l'environnement du corps (110) et pour utiliser celle-ci pour sa propre alimentation en énergie et/ou une alimentation en énergie d'autres noeuds de réseau (132).

13. Réseau (126) selon la revendication précédente, avec lequel le noeud de réseau (132) comprend au moins un capteur électrochimique (146), le capteur électrochimique (146) étant configuré pour détecter au moins une grandeur mesurée dans un mode de détection et le capteur électrochimique (146) étant en outre configuré pour obtenir de l'énergie électrochimique dans un mode d'obtention d'énergie.

14. Réseau (126) selon l'une des revendications précédentes, comprenant en outre au moins un appareil portatif (160) intégrable dans le réseau (126) et pouvant être déconnecté du réseau (126), disposant d'au moins une fonction d'affichage, notamment un appareil de mesure médical (166) et/ou un téléphone mobile (162), le réseau (126) étant configuré pour intégrer l'appareil portatif (160) automatiquement dans le réseau (126) lors d'un contact de l'appareil portatif (160) avec le corps (110) du patient (128), notamment une main (168) du patient (128).

15. Noeud de réseau (132) destiné à être utilisé dans un réseau (126) selon l'une des revendications précédentes, comprenant au moins une fonction médicale, notamment une fonction de diagnostic et/ou une fonction d'administration de médicament, le noeud de réseau (132) comprenant au moins une unité de communication pouvant être reliée avec le corps (110) du patient (128) et étant configuré pour communiquer directement avec d'autres noeuds de réseau (132) du réseau (126) par le biais du corps (110) du patient (128) et pour échanger des données et/ou des instructions, **caractérisé en ce que** le noeud de réseau (132) est configuré de telle sorte qu'au moins deux des noeuds de réseau (132) peuvent prendre en charge une commande du réseau (126).
